# EUROPEAN PATENT SPECIFICATION

(11) **EP 1 836 968 B1**
(45) Date of publication and mention of the grant of the patent: **17.09.2008**
(21) Application number: 07104166.9
(22) Date of filing: 14.03.2007
(51) Int. Cl.: A61B 17/00

(54) **Closure device**
Verschlussvorrichtung
Dispositif de fermeture

(30) Priority: 22.03.2006 US 386100
(43) Date of publication of application: 26.09.2007
(73) Proprietor: RADI MEDICAL SYSTEMS AB, 754 50 Uppsala (SE)
(72) Inventor: Preinitz, Fredrik, 753 50, Uppsala (SE); Egnelöf, Per c/o Radi Medical Systems Co., Ltd., Paklol Sub-District, Thalang District Phuket 83110 (TH)
(74) Representative: Holmberg, Nils Anders Patrik

(56) References cited:
- WO-A-96/32882
- WO-A-20/05006990
- US-A1- 2005 273 135
- US-A1- 2005 288 786
- US-B1- 6 312 446

## Description

### Field of the invention

The present invention relates generally to a medical device for closing an opening or defect in an organ within a living body, e.g. a septal defect in a heart or a percutaneous puncture in a vessel wall (such as walls in arteries or other blood vessels), and in particular to an expandable and repositionable closure device, which can be remotely maneuvered from an initial positioning configuration to a final configuration in which the opening or defect is closed.

### Background of the invention

The closing of an opening in an organ of a patient is a medical procedure that frequently has to be practiced by doctors or other trained medical personnel. The opening may be a hole created by the doctor for a specific and usually temporary purpose, or the opening can be a congenital or acquired defect. An example of the former would be a puncture hole created in a patient's femoral artery to obtain access to the coronary system, while an example of the latter is a septal defect in a patient's heart. For descriptive and illustrative purposes the present invention will be described with reference to such a septal defect, although such techniques can be applied to other fields of application, such as walls in arteries or other blood vessels.

As is well-known, the human heart is divided into four chambers: the left atrium, the right atrium, the left ventricle, and the right ventricle. The atria are separated from each other by the interatrial septum, and the ventricles are separated by the interventricular septum.

Either congenitally or by acquisition, abnormal openings or holes can form between the chambers of the heart, causing shunting of blood through the opening or hole. For example, with an atrial septal defect, blood is shunted from the left atrium to the right atrium, which produces an over-load of the right side of the heart. In addition to left-to-right shunts such as occur in patent ductus arteriosus from the aorta to the pulmonary artery, the left side of the heart has to work harder because some of the blood will recirculate through the lungs instead of going to the rest of the body. The ill effects of such lesions usually cause added strain to the heart with ultimate failure if not corrected.

One way to cure a septal defect in the septum of a heart is to position and anchor a specially designed closure device at the septum such that both sides of the septal defect are spanned by the closure device to thereby close the defect. Examples of such septal defect closure devices are known from the U.S. Patent Nos. 5,853,422; 6,024,756; 6,117,159 and 6,312,446 to Huebsch et al., which disclose a closure device comprising a cylindrical shaft of metal or polymeric material with concentric parallel cuts through the wall of the device to thereby create flattened support struts. The centers of the support struts are intended to move radially away from the longitudinal axis of the device in a hinge-like fashion in response to movements of the proximal and distal ends of the device towards the center thereof.

A similar septal defect closure device is also disclosed in US 2005/273135 to Chanduszko, which is in accordance to the preamble of claim 1.

Within the medical field it is of utmost importance that closure devices work properly, and the general object of the present invention is therefore to improve a closure device of the aforementioned type in such a way that a more reliable device is obtained, whose correct movement pattern is guaranteed such that the struts actually move radially outwards, and not move radially inwards.

### Summary of the invention

The above-mentioned object is achieved by the present invention according to the independent claim. Preferred embodiments are set forth in the dependent claims.

According to the present invention, a septal defect closure device comprises an elongated tubular member in which a first set of longitudinal slits or cuts have been made on a first side of an uncut central portion and a second set of longitudinal slits or cuts have been made on the opposite side of the central portion. On each side of the central portion, the slits extend towards the ends of the tubular member to terminate a short distance before the respective end, such that uncut proximal and distal end portions are formed. The tubular member, which is made from a flexible and preferably resorbable material, has thereby been provided with proximal and distal sets of struts or ribs. The distal ends of the distal struts are flexibly connected to the distal end portion of the tubular member, while the proximal ends of the distal struts are flexibly connected to the central portion. Similarly, the proximal ends of the proximal struts are flexibly connected to the proximal end portion of the tubular member, while the distal ends of the proximal struts are flexibly connected to the central portion. The struts are further each provided with a hinge section such that each strut in effect is divided into two articulated arms.

When the septal defect closure device during use is compressed such that the distal and proximal end portions are forced towards each other, the hinge sections of the struts move radially out from the longitudinal central axis of the closure device, and the respective arms of the struts assume an essentially perpendicular angle to the central axis of the closure device. The septal defect closure device further comprises a central elongated locking member, which can be either separate from or already integrated with the tubular member. In the former case, a unitary elongated locking member is inserted into the tubular member such that the distal end portion of the tubular member abuts one or several radially protruding portions of a distal end of the locking member, and the proximal end portion of the tubular member is then pushed over one or several radially protruding portions of a proximal end of the locking member. In the compressed state, the central, proximal and distal portions of the tubular member fit snugly along the central locking member, and the closure device is held in the compressed state by the enlarged distal and proximal rims or other radially protruding portions of the locking member, which prevents the closure device from resuming its original elongated shape. The device further comprises a keying feature, which prevents rotational movement of the locking member in relation to the tubular member.

For this particular kind of closure device it is crucial that the struts and thereby the hinge sections move outwards (and not inwards), such that an expanded closure device is provided. In accordance with the present invention, this is accomplished by arranging the two articulated arms of each strut slightly inclined with respect to each other. The arms are held at an angle by a small protrusion on the inside of one of the two hinged arms. Therefore, even in the introduction phase, when the closure device has an essentially longitudinal shape, the hinge section of each strut will be slightly further out from the central axis of the closure device than the proximal and distal ends of the struts. This angled relation between the two articulated arms of each strut ensures that the struts move outwards when the closure device is compressed and expanded by forcing the proximal and distal end portions of the closure device towards each other.

In addition, the hinge section is constructed so that upon folding of the hinge, a longitudinally slit middle section of the hinge region folds outwards, which achieves two goals. Firstly, the total diameter of the device in the expanded state is larger than the length of the two sets of struts protruding out from the center of the device, thereby adding to the total reach of the device in supporting itself on the underlying tissue. Secondly, the slit middle section provides space to insert the protrusion in the corresponding slit on the opposing arm, allowing the two arms to fully align when the device is completely expanded, thereby flattening the device against the underlying tissue to the furthest possible extent.

### Brief Description of the Drawings

Fig. 1 is a schematic illustration of a human heart having an atrial as well as a ventricular septal defect.
Fig. 2 is a schematic illustration of a human heart having a septal defect, which is to be closed by means of a medical procedure that, in a first step, involves the positioning of a septal defect closure device according to the present invention.
Fig. 3 illustrates an intermediate step in the medical procedure, in which a distal portion of the closure device of Fig. 2 is expanded in order to locate the septal defect from the distal side of the septal defect.
Fig. 4 illustrates another intermediate step in the medical procedure, in which a proximal portion of the closure device of Fig. 2 is expanded in order to locate the septal defect from the proximal side of the septal defect.
Fig. 5 illustrates the completion of the medical procedure, wherein the closure device of Fig. 2 has been secured in the septum surrounding the septal defect.
Fig. 6 shows a closure device according to the invention in a positioning configuration before the longitudinal compression of the closure device.
Fig. 7 shows the closure device of Fig. 6 in an intermediate semi-compressed state.
Fig. 8 shows a locking member which constitutes a separate part of a closure device.
Fig. 9 shows the closure device of Fig. 6 locked in a final compressed and locked state.

### Detailed Description of Preferred Embodiments

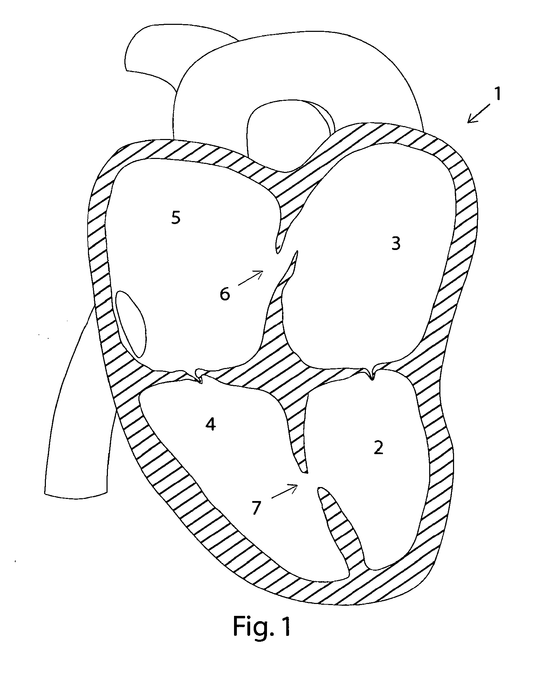

A schematic cross-sectional view of a human heart 1 is shown in Fig. 1. The heart 1, with its left ventricle 2, left atrium 3, right ventricle 4, and right atrium 5, suffers from an atrial septal defect 6 as well as a ventricular septal defect 7. Below a medical procedure will be discussed in which an atrial septal defect is closed. It should, however, be clear that a septal defect closure device according to the present invention equally well could be employed to close a ventricular septal defect such as ventricular septal defect 7 of Fig. 1. It should further be noticed that the septal defects 6, 7 can be accessed from different vessels, e.g. from the superior or inferior vena cava, or from the aorta. This implies, in turn, that throughout the present description terms like "distal" and "proximal" should always be seen from the end of a delivering catheter, through which a septal defect closure device is delivered (and not from any particular chamber or vessel of a heart).

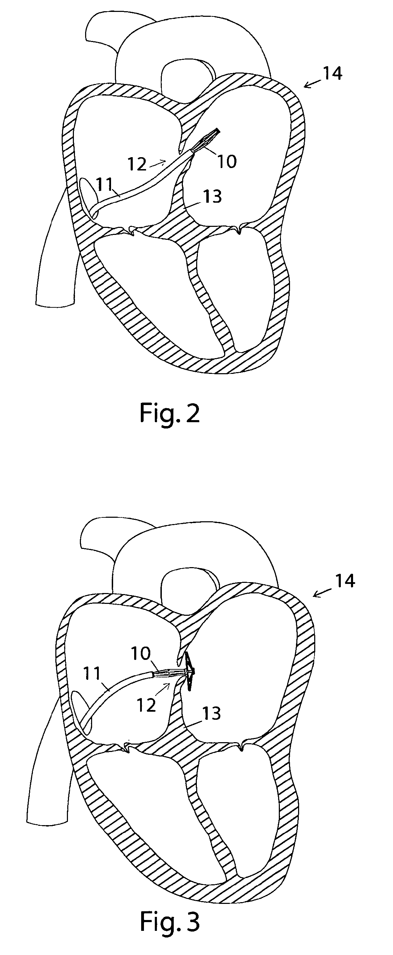

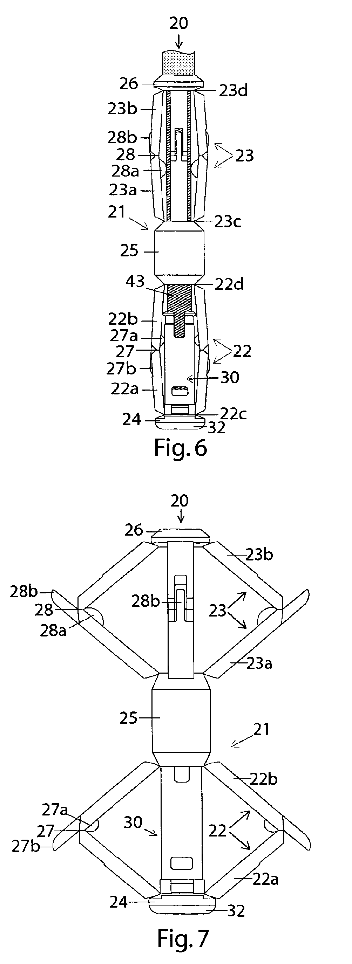

In conjunction with Figs. 2 to 5, a medical procedure will be briefly described, in which a septal defect closure device according to one embodiment of the present invention is employed to close a septal defect in the septum of a heart; and thereafter different configurations and parts of the closure device itself will be described in detail in conjunction with Figs. 6 to 9.

Fig. 2 illustrates a septal defect closure device 10 according to the present invention, which has been introduced into an atrial septal defect 12 in the atrial septum 13 of a heart 14 using a delivering catheter 11. The closure device 10 is of the same general construction that has been generally described above, and comprises an elongated tubular member in which distal and proximal sets of struts have been provided. The distal struts extend from a central portion of the closure device 10 to a distal end portion thereof, and the proximal struts extend from a proximal end portion of the closure device 10 to the central portion. As already discussed, each strut is provided with a hinge region, and each strut is thereby effectively divided into two hinge-connected arms. The characterizing details of the hinge region will be discussed further below. In Fig. 2, the closure device 10 is shown in an initial positioning configuration, in which the arms of each strut are substantially - but not perfectly - aligned with each other. In this uncompressed positioning configuration, the closure device 10 therefore has a generally elongated tubular shape, which facilitates the introduction of the closure device 10 into the artery and heart of a patient. As indicated above and also previously discussed, a special feature of the closure device 10 is that the arms of each strut exhibit a small angle in relation to each other, something that will be thoroughly explained below. Here, it suffices to say that this angle exists also in the introduction phase; and it should therefore be understood that the above statement saying that the closure device 10 in this state has a generally tubular shape should not be taken literally but seen from a practical point of view.

To ascertain correct positioning of the closure device 10 with respect to the septal defect 12, the distal set of struts can be moved radially outwards from the central axis of the closure device 10, such that a partly expanded configuration is obtained. The radial movements of the distal struts are effectuated by partially compressing the closure device 10 through the maneuvering of a mechanical actuator (not shown in the figures). In this semi-expanded locating configuration, the closure device 10 is retracted until the distal struts abut the distal side of the atrial septum 13 surrounding the septal defect 12. The septal defect 12 can thereby be located by a doctor, who in this phase of the medical procedure will feel a marked increase in resistance against further retraction. This intermediate step of the medical procedure is depicted in Fig. 3.

A special advantage of the present invention is significant in the intermediate step illustrated in Fig. 3. Without the measures according to the invention, the arms of each strut would initially be perfectly aligned with each other; and when the distal portion of the closure device is longitudinally compressed to achieve the configuration shown in Fig. 3, there is a risk that some of the struts instead bend inwards. By seeing to that the two arms of each distal strut are arranged such that they are not perfectly aligned with each other, but exhibit a small inclination, it is ensured that each arm moves outwards, and the closure device will therefore assume the proper configuration as illustrated in Fig. 3. The angled arrangement of the two arms of each distal strut is achieved by a new and improved design of the hinge region, which will be further described below.

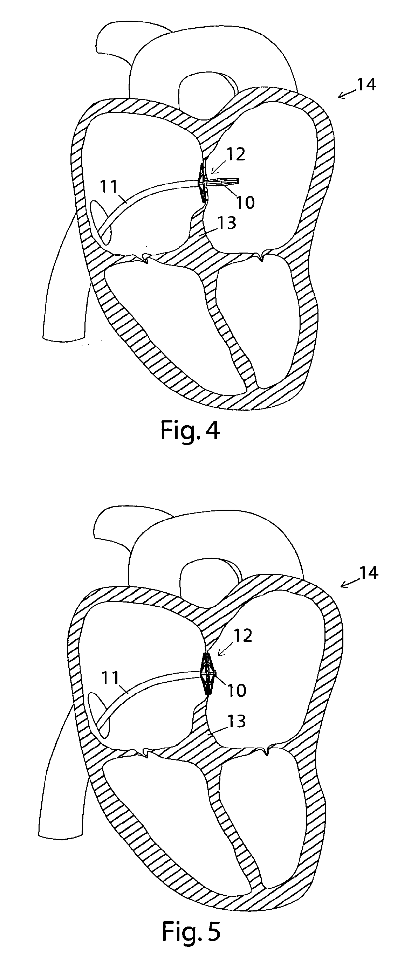

As an alternative or complement, the proximal set of struts can be moved radially outwards from the central axis of the closure device 10, such that another partly expanded configuration is obtained. As before, the radial movements of the proximal struts are accomplished by partially compressing the closure device 10 through the maneuvering of the mechanical actuator mentioned above. In this second semi-expanded locating configuration, either the closure device 10 is advanced out of the catheter 11, or the entire assembly is advanced, until the proximal struts abut the proximal side of the atrial septum 13 surrounding the septal defect 12. The septal defect 12 can thereby be located by a doctor who in this phase of the medical procedure will feel a marked increase in resistance against further advancement. This intermediate step of the medical procedure is depicted in Fig. 4. It should be mentioned that the closure device 10 can be reversibly moved between the elongated tubular positioning configuration of Fig. 2 and either of the intermediate configurations shown in Fig. 3 and Fig. 4, respectively.

Also when the proximal portion of the closure device 10 is compressed to achieve the configuration shown in Fig. 4, the present invention provides the same advantage. By seeing to that the two arms of each distal strut are arranged such that they are not perfectly aligned with each other, but exhibit a small inclination, it is ensured that each arm moves outwards, and the closure device will therefore assume the proper configuration as illustrated in Fig. 4. In fact, especially when moving back and forth between the different intermediate configurations, it is of utmost importance that the risk of inverting the hinge regions is minimized.

When the atrial septum 13 and thereby the septal defect 12 have been correctly located, either by the step shown in Fig. 3 or by the step of Fig. 4, or by both steps, the closure device 10 is fully expanded such that the proximal struts as well as the distal struts are forced radially outwards by maneuvering of the mechanical actuator mentioned above. In this closing configuration, the closure device 10 spans both the distal side and the proximal side of the septal defect 12, and is then held in this position. As can be seen in Fig. 5, the closure device 10 sandwiches the atrial septum 13 to thereby close the septal defect 12 therein. It should be mentioned that the term "close" or similar terms used herein in conjunction with the description of the closing of a septal defect should not be taken too literally. Such terms are meant to encompass all stages from actually sealing or closing off a septal defect to merely restricting the flow of blood therethrough, the important feature being that the closure device permits and facilitates healing of the septal (or other type of) defect over time. To improve the sealing capability of a closure device of the present type, it is conceivable that the distal and/or proximal struts at least partly are covered by a thin membrane or formed integrally with a thin membrane, which preferably is made from a resorbable material. This feature is further described in connection with the detailed description of the struts.

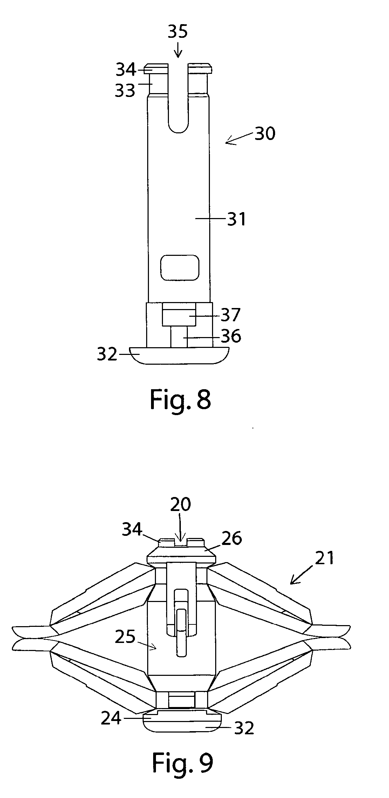

An embodiment of a septal defect closure device 20 according to the present invention is illustrated in Fig. 6. Fig. 6 shows the closure device 20 in a first or positioning configuration in which the closure device 20 has the general shape of an elongated, essentially tubular member 21, through which a number of longitudinal, parallel cuts or slits have been made to thereby form a first or distal set of struts 22 and a second or proximal set of struts 23. The first strut set 22 extends between a first end portion 24 of the tubular member 21 and a central portion 25 thereof, while the second strut set 23 extends between the central portion 25 and a second end portion 26 of the tubular member 21. The first and second end portions 24, 26 as well as the central portion 25 are uncut and are preferably shorter than the slit portions of the tubular member 21. Somewhere along the length of the first set of struts 22, the tubular member 21 has been provided with a hinge region 27, which will thereby act as a hinge or articulation 27, effectively dividing each strut 22 into two articulated arms: a first or distal arm 22a and a second or proximal arm 22b. Similarly, the struts in the second set of struts 23 are each provided with a hinge section 28, which in effect divides each strut 23 into two articulated arms: a first or distal arm 23a and a second or proximal arm 23b. In Fig. 6, the device has been provided with two distal arms 22 and four proximal arms 23, however the number of arms for each set can be varied without departing from the scope of the present invention, as will be further described below. In addition, the closure device 20 also comprises a locking member 30, which is further described below in connection with Fig. 8.

To facilitate the expanding movement of the closure device 20, each arm 22a, 22b, 23a, 23b can at the end that is opposite to the central hinge sections 27, 28 be provided with a recess or weakened section, which acts as a hinge, similarly to the hinge sections 27, 28. In Fig. 6, the distal arms 22a of the first strut set 22 are provided with hinge sections 22c, while the proximal arms 22b have hinge sections 22d; and the distal arms 23a of the second strut set 23 are provided with hinge sections 23c, while the proximal arms 23b have hinge sections 23d. In effect, each arm 22a, 22b, 23a, 23b is provided with two articulation points. It should also be mentioned that if the struts are made from a material being flexible enough, it is possible that no extra hinge sections, such as hinge sections 22c, 22d, 23c, 23d, are provided as the material in itself can act as an articulation or a hinge. Also in the latter case, each arm is, however, effectively provided with two articulation points.

A special feature of the present invention should be clearly visible in Fig. 6, where it can be seen that the first and second strut sets 22, 23 are arranged such that the hinge sections 27, 28 are further out from the longitudinal central axis of the closure device 20 than the first end portion 24, the central portion 25, and the second end portion 26. The arms 22a, 22b of the first strut set 22 have thereby assumed an outwardly angled relation to each other; and the arms 23a, 23b of the second strut set 23 have a similar outwardly angled relation to each other. This angled relation is achieved by the specific design of the hinged region, which is described in detail below. It should however be noted that it is within the scope of the present invention that either the first arms 22a, 22b or the second arms 23a, 23b exhibit such a positive deviation from a perfect alignment. The angled relation of the arms of a strut set is characterized by a tilt angle which is positive when the arms of a strut set point slightly outwards from the central axis of a tubular member (one example of a positive tilt angle is shown in Fig. 6). The provision of a tilt angle should be guaranteed in the positioning configuration of a closure device, i.e. when a strut set is outside a delivering catheter and before any longitudinal compression of this strut set by a mechanical actuator. This tilt angle, which can be regarded as a deviation from a perfect alignment (straight line), can range from 1 ° to 10°, and more preferably from 3° to 7°. The tilt angle is defined as the angle under which a first line extending through the two articulation points of a first arm of a strut set intersects with a second line extending through the two articulation points of a second arm of this strut set. It is particularly important to keep this definition in mind when - in order to create hinge points for a strut arm - material is removed from opposite sides at the two ends of the arm. In this latter case, the struts could actually appear to be aligned along a straight line, but as long as the articulation points of the arms of the struts are not aligned, a tilt angle is provided in accordance with the definition above (with the tilt angle being positive when the articulation point(s) where the two arms meet are further away from the longitudinal central axis than the articulation points at each end of each strut set). The provision of a tilt angle should also be guaranteed in the possible intermediate configurations of a closure device, i.e. when a strut set has been longitudinally compressed and thereafter returned to the original elongated state.

According to the present invention, the hinge region has been constructed so as to comprise a middle longitudinally extended section which will constitute an extra protruding arm 27b when folded, which is illustrated in Fig. 6 and also in Fig. 7, which is further described below. This protruding arm 27b can be of a variety of lengths, but is preferably approximately one third of the distance from the hinge region 27 to the connection point 22c of the arm at the distal end portion 24. On the inside of the proximal arm 22b there is also a protrusion or knob 27a adjacent to the hinge, which serves to keep the two connected arms 22a, 22b at an angle towards each other even in the initial positioning configuration of the tubular member, as described above. As illustrated in Fig. 6, this protrusion encounters either the central cylindrical holder member 43 or the locking member 30, if present, in the initial configuration of the arms, which prevents the two connected arms from aligning completely with each other. As can be seen in Fig. 6, the hinge section 28 is constructed as the mirrored arrangement of hinge section 27, with two longitudinal cuts creating an extra protruding arm 28b on distal arm 23a and a protrusion 28a encountering the central holder rod 43 in the elongated state. It should however be noted that it is within the scope of the present invention that either hinge region 27 or hinge region 28 comprises the abovementioned hinge construction, and that the other central hinge region can thereby be constructed by simply removing material on the outer side of the struts, thus creating weakened sections that will act as hinges.

It is should further be emphasized that the term "tubular" is merely intended to indicate the general shape of an elongated, hollow member, which comprises a number of struts, the ends of which are connected to ring-shaped or essentially cylindrical members, and which in a first positioning configuration assumes an essentially tubular shape. In other words, a tubular member, like tubular member 21, does not actually have to be cut or slit in order to create distal and proximal struts and the hinge regions described above. On the contrary, a tubular member, having struts with hinge regions constructed as described above, or weakened hingesections, as well as ring-shaped central, distal and proximal end portions, can advantageously be directly produced in this form, e.g. by injection molding or die casting.

In Fig. 7, the closure device 20 of Fig. 6 is depicted in a semi-expanded state in which the distal and proximal end portions 24, 26 of the closure device 20 have been moved towards the central portion 25. The hinge sections 27, 28 of the first and second struts 22, 23 have thereby been forced to move outwards from the central axis of the closure device 20, and the articulated arms 22a, 22b and 23a, 23b have assumed a more angled relation to the central axis of the closure device 20 than that of the elongated initial configuration. Here it should be recognized that the configuration shown in Fig. 7 partly is for illustrative purposes; in practice either of the two end portions 24, 26 could be moved towards the central portion 25, to assume the locating configurations shown in Fig. 3 and Fig. 4, respectively. The semi-expanded configuration of Fig. 7 could, however, also be used to determine the proper position for the closure device 20, and can also be regarded as a configuration prior to a fully closed configuration described below in conjunction with Fig. 9. With reference to Figs. 6 and 7, the present invention can be summarized as guaranteeing that the transition from the initial configuration shown in Fig. 6 to the semi-expanded state of Fig. 7 actually takes place without any difficulties. It should also be noted that the protruding middle part 27b, 28b of each hinge region effectively extends the reach of the device over a larger area of the septum, facilitating the proper placement of the closure device according to the present invention.

As can be seen in Figs. 6 and 7, the closure device 20 comprises further a locking member 30, which is separately illustrated in Fig. 8. The locking member 30, which according to the invention constitutes a separate part of closure device 20, comprises a hollow tubular body, which along the central part of its length is provided with an outer diameter approximately equivalent to the inner diameter of the tubular member 21 of the closure device 20. More specifically, the locking member 30 comprises a distal end rim 32, an intermediate body 31, a proximal groove 33, and a proximal end rim 34. The distance between the distal end rim 32 and the proximal end rim 34 is considerably smaller than the length of the tubular member 21. As the observant reader already may have appreciated, the diameter of the distal end rim 32 is larger than the inner diameter of the distal end portion 24 of the tubular member 21. In turn, the inner diameter of the distal end portion 24 is marginally larger than the diameter of the intermediate portion 31 of the locking member 30, such that the distal end portion 24 of the tubular member 21 can slide over the locking member 30 until the distal end portion 24 abuts the distal end rim 32. In this position, as seen in Figs. 6 and 7, locking knob 37 functions by locking the distal end portion 24 against the distal end rim 32, such that the tubular member and the locking member are locked at their distal ends. In addition, to prevent rotational movement of the tubular member 21 relative to the locking member 30, a keying feature between the locking member and the tubular member is provided. In one embodiment, shown in part in Fig. 8, the locking member is supplied with a protrusion 36 which is matched by a recess on the inner surface of distal end portion 24 (not shown in the figures).

Similarly to the inner diameter of distal end portion 24, the inner diameters of the central portion 25 and the proximal end portion 26 of the tubular member 21 are marginally larger than the outer diameter of the intermediate portion 31 of the locking member 30. Additionally, the outer diameter of the proximal end rim 34 is slightly larger than the inner diameter of the proximal end portion 26. During use, the proximal end portion 26 of the tubular member 21, which is made from a somewhat elastic material, must therefore be forced over the proximal end rim 34 and can then slide on the intermediate portion 31. As can be seen in Fig. 8, the locking member 30 preferably comprises a recess 35, which in combination with the groove 33 provide the proximal end rim 34 with certain resilience, facilitating the sliding of the proximal end portion 26 of the closure device 20 over the proximal end rim 34 of the locking member 30.

In the embodiment of the locking member shown in Fig. 8 the distal and proximal ends 32, 34 comprise a completely circumferential circular rim and a discontinuous circumferential rim, respectively. It should be noted that the locking member's distal and proximal ends can comprise other shapes, provided that the distal end 32 supplies a point of hindrance for the distal end portion 24 and that the proximal end 34 both allows proximal end portion 26 to be forced over it and locks the final expanded state of the device in place. Many shapes are conceivable such that at least one outer cross-sectional dimension of the distal end 32 is larger than at least one inner cross-sectional dimension of the end portion 24 and at least one outer cross-sectional dimension of the proximal end 34 is adapted to at least one inner cross-sectional dimension of the end portion 26. One such possibility is to supply the ends with one or several radial protrusions.

In Fig. 9, the closure device 20 is shown in a closed and locked state, in which the distal and proximal end portions 24, 26 of the tubular member 21 have been fully moved towards each other until the central portion 25 is positioned over the intermediate portion 31 of the locking member 30 and the proximal end portion 26 has been moved over the proximal end rim 34 of the locking member 30. The closure device 20 is held in this compressed state due to the enlarged distal and proximal end rims 32, 34 of the locking member 30, which have diameters larger than the distal end portion 24 and the proximal end portion 26, respectively.

In Figs. 6, 7 and 9, the central portion 25 of the tubular member 21 has been depicted with a longitudinal length larger than the lengths of the distal and proximal end portions 24, 26. The length of a central portion of a tubular member can, however, be varied, e.g., be equivalent in length to the end portions, and can in particular be adjusted to the thickness of a particular septum at which the corresponding closure device is to be placed. In addition, with reference to Fig. 9, a person skilled in the arts will realize that changing the relative length of the central portion to the struts will also affect the angle of the arms in the final expanded state of the closure device. Furthermore, the outer diameter of the central portion can be adapted to varying dimensions of the opening which is to be sealed by the device, provided that the inner diameter of the central portion is maintained such that the locking member can slide through the tubular member, and that the actuator member (a cylindrical member, not shown in the figures, used to place the device) can accommodate the device in its initial tubular configuration. The alteration of the central portion's diameter can e.g. be achieved by changing the thickness of the material in the central portion.

Another way to facilitate the adaptation of a septal defect closure device to septa having different thicknesses is to arrange the distal set of struts and the proximal set of struts as two separate members. Such an arrangement would effectively correspond to cutting a tubular member like the tubular member 21 of Fig. 6 into two separate tubular members. These two tubular members would then independently of each other be movable along the length of a common locking member similar to locking member 30 of Fig. 8. Also in this embodiment, at least one of the two tubular members would comprise arms that are connected with the hinge region defined above, such that an angled relation is achieved even in the initial elongated configuration of the struts.

The septal defect closure device has been shown with proximal and distal struts having equal lengths. It is, however, possible to provide a closure device having proximal struts with one length and distal struts with a different length. It may, for example, be desirable to arrange a closure device in such a way that the left part of the closure device, i.e. the part that is implanted into the left atrium of a heart, is smaller than the right part of the closure device, to thereby reduce the amount of artificial material introduced into the left atrium, which in turn may reduce the formation of thrombogenic material therein. In this context, it should be recognized that it is not mandatory that a heart is accessed via the venous system, as is shown in Figs. 2 to 5, but the heart could be accessed via the arterial side. This implies that if a doctor wishes to place a smaller part of a closure device at the left side of a heart than at the right side of the heart, then this smaller part (i.e. the shorter struts) will constitute the distal set of struts if the heart is accessed via the venous system, whereas the smaller part will constitute the proximal set of struts if the heart is accessed through the arterial system.

As mentioned earlier, it is conceivable that the distal and/or proximal struts at least partly are covered by a thin membrane or formed integrally with a thin membrane, which preferably is made from a resorbable material. This membrane can be e.g. a thin film made from resorbable polymers or a mesh made from electrospun polymers, which are, for example, woven or braided together. Similarly to the situation discussed above with a smaller part of the device placed in the left atrium of the heart, the membrane is preferably provided on the right side of the septal defect, to minimize the amount of material introduced into the left side of the heart. A thin membrane is also particularly beneficial when used for the repair of a vessel wall, where the membrane is preferably placed inside the vessel in order to take advantage of the blood pressure in keeping the device in place.

An advantage of the present invention is that the final expanded size of the two different sides of the closure device can additionally be controlled independently of the length of the individual struts, thereby not affecting the initial length of the tubular member. This is achieved by selecting a different length of the protruding arm in the hinge regions (27b and 28b, respectively, in Figs. 6 and 7). This length can essentially range between zero and a length corresponding to the length of the outermost arms (22a and 23b) of each respective set of struts. However, when using a protruding arm 27b, 28b shorter than the width of the corresponding protrusion 27a, 28a, an excision must be made which allows the insertion of the protruding knob 27a, 28a into the aperture in the opposing arm 22a, 23b to allow the two arms to meet when the hinge is fully folded. A longer length though, will effectively increase the reach of the expanded device on the underlying tissue, without increasing the initial size of the tubular member.

It has already been mentioned that the length of the distal struts can differ from the length of the proximal struts; and it is also possible to have different lengths of the articulated arms within a strut set, such that, for example, the distal arms are longer than the proximal arms, or vice versa. The arms, or more specifically, the lengths that actually contact a septum or a vessel wall, e.g. from the central hinge regions 27, 28 to the point 22d, 23c connecting to the central portion 25, can, for example, be shorter than the arms that do not contact the septum or the vessel wall, to thereby ensure a reliable closing of a septal defect in the septum or a puncture hole in the vessel wall. Obviously, the length of the protruding sections 27b, 28b of the arms contacting the septum can however be selected independently of the length from the central hinge regions 27, 28 to the point connecting to the central portion 25, thereby ensuring a suitable size of contact area of the closure device on the septum.

In the embodiment shown in Figs. 6, 7 and 9 the closure device is shown with two distal struts 22 and four proximal struts 23. However, it is within the scope of the invention to use any number of struts per set, preferably between 1 and 10 struts per set, more preferably between 2 and 6 struts per set. Being able to choose the specific number of struts further increases the possibility to adapt an individual closure device to the unique anatomy of the defect to be closed. For instance, using 2 radially opposing struts at one end of the device will permit inclining of the device against the septum more easily than with additional struts. On the other hand, a larger number of struts increase the stability and number of contact points of the device on the septum.

As already has been stated, a closure device comprising a central locking member that is separate from a tubular member can be regarded as a prerequisite for other advantageous effects. A two-piece closure device is generally easier and thereby cheaper to manufacture. If, for example, the closure device is produced by injection molding, the molds - i.e. one mold for the locking member and one mold for the tubular member - can be given comparatively less complicated shapes than if the closure device was to be molded in a single mold.

It is in particular anticipated that a locking member is made from a first material and that a tubular member is made from a second material, something which in practice may require that the locking member is separate from the tubular member. With different materials some specific advantages can be achieved. If, for example, the closure device is a resorbable closure device, then the resorption time of the material in the locking member can be different from the resorption time of the material in the tubular member, such that the mechanical properties of the closure device are maintained until the surrounding tissue has healed to the point where the support of the closure device is not necessary anymore. Further, whether or not the materials are resorbable materials, different requirements are put on the different pieces. For example, the material in the hinge portions of a tubular member must be ductile and have a high durability, whereas the locking member must have a rather high stiffness. Furthermore, it may be necessary to have one material in a locking member and another material in a tubular member in order to match the resorption times due to different dimensions of the members involved in a resorbable closure device.

The closure device according to the present invention is preferably made form a resorbable material. Examples of resorbable materials for the tubular member and the locking member may include, but are not limited to, those materials made from aliphatic polyesters, polyether esters, and polycarbonates. More specifically, synthetic resorbable polymers such as homopolymers and copolymers made from any of the monomers lactide, glycolide, epsilon-caprolactone, trimethylene carbonate, and paradioxanone are advantageous because of their long clinical use. Other lactones that may be used together with any of the abovementioned monomers to make copolymers of various properties are valerolactone, b-butyrolactone and dioxepanone, however also other 4, 5, 6 and 7 member lactones may be of interest to obtain the characteristic material properties needed to fulfill a smooth operation of the invented closure device.

The tubular member could preferably be made from a semi-crystalline material with a lower modulus than the locking member. As previously stated, the device could, e.g. because of the hinge portions, have a more flexible material in the tubular member. Such material is preferably made from a block copolymer characterized by having a soft middle part distinguished by having a glass transition temperature below body temperature and a semi-crystalline part at each end of the soft middle part. The semi-crystalline part could be polymerized from any of the monomers glycolide, lactide, or paradioxanone. Since polyparadioxanone is a relatively soft and pliable material compared to polyglycolide and polylactide, the tubular member can be made from pure polyparadioxanone itself.

The locking member can be made from any of the above materials, but to secure the locking mechanism it is advantageous if the material is stiffer than the material used in the tubular member. The material should also preferably resorb at a somewhat slower pace than the tubular member. The locking member could also be made from amorphous or semi-crystalline material, and preferably from homopolymers or copolymers where the main monomer component is lactide, caprolactone, or paradioxanone.

Further examples of synthetic resorbable polymers that may be used in the tubular or locking member of the invented closure device are resorbable polymers made from dicarboxylic acids such as succinic, glutaric, adipic or pimelic acids with various forms of diols, polymers composed of segmented blocks of polyethyleneglycol and butyleneterephthalate, various forms of tyrosine carbonate polymers, phosphazene polymers, orthoester polymers or resorbable polyurethanes.

A particular advantage of the groups of synthetic resorbable polymers mentioned above is that various mechanical properties can be accomplished by simply changing the monomer composition in the homopolymer or copolymer. Further, in contrast to natural biopolymers, these materials can be molded and machined into complex structures, and by varying the monomer composition large time spans can be achieved for their resorption times.

It may be appreciated that it can be advantageous to provide a radiopaque closure device which is visible in an X-ray machine. When the closure device is made from a synthetic resorbable polymer, a radiopaque closure device can conveniently be produced by mixing the polymer with a suitable radiopaque agent. A suitable radiopaque agent is barium sulfate, which can be blended into the polymer or copolymer in an amount between 5% and 50%, and more preferably in an amount of 15% to 30%, to obtain the opacity needed in order to locate the closure device during an X-ray observation. Radiopaque materials can be used in a tubular member of the closure device, but is preferably used in the locking member, which marks the center of the device. The radiopaque agent, e.g. barium sulfate, can be - instead of being mixed with the polymer - introduced into preformed holes in the closure device, which are then sealed by a synthetic resorbable material. As an alternative, preformed holes can be plugged with a resorbable material containing a large amount of a radiopaque agent, e.g. barium sulfate.

Although the present invention has been described with reference to specific embodiments, also shown in the appended drawings, it will be apparent for those skilled in the art that many variations and modifications can be done within the scope of the invention as described in the specification and defined with reference to the claims below. The arms, struts and hinge regions need not have the shape shown in the drawings. It is possible to have different lengths of the articulated arms within a strut set, such that, for example, the distal arms are longer than the proximal arms, or vice versa. The shape and design of the hinges can be varied within the scope of the claims.

## Claims

1. Medical closure device having a longitudinal central axis, comprising:
a tubular member (21) having a length and a first set of struts (22) extending between a first end portion (24) and a central portion (25) and a second set of struts (23) extending between said central portion and a second end portion (26),
each strut being provided with a hinge section (27, 28) that can act as a hinge, to thereby effectively divide each strut into two arms, such that said closure device is movable between a first elongated configuration and a second configuration in which the first and second end portions have been moved towards each other such that said hinge sections of the first and second sets of struts have moved radially away from said longitudinal central axis,
**characterized in that**
the two arms of at least one of the sets of struts, in the first configuration, are arranged with a positive tilt angle to each other,
said tilt angle is achieved by a protrusion on the inside of said set of struts adjacent to said hinge sections, where at least one protrusion is adapted to
contact a central holder rod (43) of a mechanical actuator or a locking member (30) within said tubular member (21) when in the first elongated configuration, to thereby ensure that the second configuration can be achieved.

2. The medical closure device according to claim 1, **characterized in that** said tilt angle ranges from 1° to 10°.

3. The medical closure device according to claim 1, **characterized in that** at least one of the first and second sets of struts comprises between 1 and 10 struts.

4. The medical closure device according to claim 1, **characterized in that** at least one of the first and second sets of struts is at least partly covered by a membrane.

5. The medical closure device according to claim 1, **characterized in that**
one arm of at least one set of struts comprises a longitudinally extended section (27b, 28b) originating essentially from said hinge section, and said extended section is essentially parallel to the arms in the initial introduction configuration, and
said extended section will fold radially outwards when the device is compressed.

6. The medical closure device according to claim 1, **characterized in that** the closure device further comprises a locking member (30), which has
a first end (32) with at least one outer cross-sectional dimension larger than at least one inner cross-sectional dimension of the first end portion (24) and a second end (34) with at least one outer cross-sectional dimension larger than at least one inner cross-sectional dimension of the second end portion (26), and
a distance between the first and second ends which is smaller than the length of the tubular member, such that, when the locking member is positioned in the tubular member such that the first end of the locking member abuts the first end portion, the second end portion can be forced over the second end such that the closure device is held in an expanded configuration in which the first and second sets of struts have moved radially away from said longitudinal central axis.

7. The medical closure device according to claim 1, **characterized in that** the closure device at least partly is made from a synthetic resorbable polymer.

8. The medical closure device according to claim 6, **characterized in that** said locking member is made from a first material and said tubular member is made from a second material.

9. The medical closure device according to claim 1, **characterized in that** the closure device comprises a radiopaque agent.

10. The medical closure device according to claim 1, **characterized in that** the closure device is adapted for closing a septal defect in a heart.

11. The medical closure device according to claim 1, **characterized in that** the closure device is adapted for closing a puncture in a vessel wall.

## Patentansprüche

1. Medizinische Verschlussvorrichtung, die eine Längsmittelachse hat, aufweisend:
ein röhrenförmiges Element (21) mit einer Länge und einem ersten Satz von Streben (22), der sich zwischen einem ersten Endabschnitt (24) und einem Mittelabschnitt (25) erstreckt, und einem zweiten Satz von Streben (23), der sich zwischen dem Mittelabschnitt und einem zweiten Endabschnitt (26) erstreckt,
wobei jede Strebe mit einer Scharniersektion (27, 28) versehen ist, die als ein Scharnier wirken kann, um dadurch jede Strebe wirksam in zwei Arme zu teilen, so dass die Verschlussvorrichtung bewegbar ist zwischen einer ersten, länglichen bzw. gestreckten Konfiguration und einer zweiten Konfiguration, in welcher der erste und der zweite Endabschnitt zueinander hin bewegt worden sind, sodass sich die Scharniersektionen des ersten und des zweiten Satzes von Streben radial weg von der Längsmittelachse bewegt haben,
**dadurch gekennzeichnet, dass**
die zwei Arme wenigstens eines der Sätze von Streben in der ersten Konfiguration mit einem positiven Neigungswinkel zueinander angeordnet sind,
wobei der Neigungswinkel durch einen Vorsprung auf der Innenseite des Satzes von Streben, angrenzend an die Scharniersektionen, erreicht wird, wobei wenigstens ein Vorsprung angepasst ist, um eine mittige Halterstange (43) eines mechanischen Stellantriebs oder eines Verriegelungselements (30) innerhalb des röhrenförmigen Elements (21) in der ersten, länglichen bzw. gestreckten Konfiguration zu berühren, um dadurch sicherzustellen, dass die zweite Konfiguration erreicht werden kann.

2. Medizinische Verschlussvorrichtung nach Anspruch 1, **dadurch gekennzeichnet, dass** der Neigungswinkel von 1° bis zu 10° reicht.

3. Medizinische Verschlussvorrichtung nach Anspruch 1, **dadurch gekennzeichnet, dass** wenigstens einer von dem ersten und dem zweiten Satz von Streben zwischen 1 und 10 Streben umfasst.

4. Medizinische Verschlussvorrichtung nach Anspruch 1, **dadurch gekennzeichnet, dass** wenigstens einer von dem ersten und dem zweiten Satz von Streben wenigstens teilweise von einer Membran bedeckt ist.

5. Medizinische Verschlussvorrichtung nach Anspruch 1, **dadurch gekennzeichnet, dass**
ein Arm wenigstens eines Satzes von Streben eine in Längsrichtung erweiterte bzw. verlängerte Sektion (27b, 28b) umfasst, die im Wesentlichen aus der Scharniersektion entspringt, und
die erweiterte bzw. verlängerte Sektion in der anfänglichen Einführungskonfiguration im Wesentlichen parallel zu den Armen ist, und
sich die erweiterte bzw. verlängerte Sektion nach außen falten wird, wenn die Vorrichtung zusammengedrückt wird.

6. Medizinische Verschlussvorrichtung nach Anspruch 1, **dadurch gekennzeichnet, dass** die Verschlussvorrichtung ferner ein Verriegelungselement (30) umfasst, dass
ein erstes Ende (32) mit wenigstens einer äußeren Querschnittsabmessung, die größer ist als wenigstens eine innere Querschnittsabmessung des ersten Endabschnitts (24), und ein zweites Ende (34) mit wenigstens einer äußeren Querschnittsabmessung, die größer ist als wenigstens eine innere Querschnittsabmessung des zweiten Endabschnitts (26), und
einen Abstand zwischen dem ersten und dem zweiten Ende, der kleiner als die Länge des röhrenförmigen Elements ist, aufweist, so dass, wenn das Verriegelungselement in dem röhrenförmigen Element derart angeordnet ist, dass das erste Ende des Verriegelungselements an den ersten Endabschnitt anstößt, der zweite Endabschnitt über das zweite Ende gedrängt werden kann, so dass die Verschlussvorrichtung in einer ausgefahrenen Konfiguration gehalten wird, in der sich der erste und der zweite Satz von Streben radial weg von der Längsmittelachse bewegt haben.

7. Medizinische Verschlussvorrichtung nach Anspruch 1, **dadurch gekennzeichnet, dass** die Verschlussvorrichtung wenigstens teilweise aus einem synthetischen resorbierbaren Polymer hergestellt ist.

8. Medizinische Verschlussvorrichtung nach Anspruch 6, **dadurch gekennzeichnet, dass** das Verriegelungselement aus einem ersten Material hergestellt ist und das röhrenförmige Element aus einem zweiten Material hergestellt ist.

9. Medizinische Verschlussvorrichtung nach Anspruch 1, **dadurch gekennzeichnet, dass** die Verschlussvorrichtung ein strahlungsundurchlässiges Agens aufweist.

10. Medizinische Verschlussvorrichtung nach Anspruch 1, **dadurch gekennzeichnet, dass** die Verschlussvorrichtung angepasst ist, einen Septumdefekt in einem Herz zu verschließen.

11. Medizinische Verschlussvorrichtung nach Anspruch 1, **dadurch gekennzeichnet, dass** die Verschlussvorrichtung angepasst ist, eine Punktion in einer Gefäßwand zu verschließen.

## Revendications

1. Dispositif médical de fermeture ayant un axe central longitudinal, comprenant :
un élément tubulaire (21) ayant une longueur et un premier ensemble d'entretoises (22) s'étendant entre une première partie d'extrémité (24) et une partie centrale (25) et un second ensemble d'entretoises (23) s'étendant entre ladite partie centrale et une seconde partie d'extrémité (26),
chaque entretoise étant dotée d'une section d'articulation (27, 28) qui peut servir d'articulation pour diviser ainsi efficacement chaque entretoise en deux bras, de sorte que ledit dispositif de fermeture est mobile entre une première configuration allongée et une seconde configuration dans laquelle les première et seconde parties d'extrémité ont été déplacées l'une vers l'autre de sorte que lesdites sections d'articulation des premier et second ensembles d'entretoises s'éloignent radialement dudit axe central longitudinal, **caractérisé en ce que :**
les deux bras d'au moins l'un des ensembles d'entretoises, dans la première configuration, sont agencés avec un angle d'inclinaison positif l'un par rapport à l'autre,
ledit angle d'inclinaison est obtenu par une saillie à l'intérieur dudit ensemble d'entretoises adjacent auxdites sections d'articulation, où au moins une saillie est adaptée pour entrer en contact avec une tige de support central (43) d'un actionneur mécanique ou d'un élément de blocage (30) à l'intérieur dudit élément tubulaire (21) lorsqu'il est dans la première configuration allongée, pour garantir ainsi que la seconde configuration peut être obtenue.

2. Dispositif médical de fermeture selon la revendication 1, **caractérisé en ce que** ledit angle d'inclinaison est de l'ordre de 1° à 10°.

3. Dispositif médical de fermeture selon la revendication 1, **caractérisé en ce qu'**au moins l'un des premier et second ensembles d'entretoises comprend entre 1 et 10 entretoises.

4. Dispositif médical de fermeture selon la revendication 1, **caractérisé en ce qu'**au moins l'un des premier et second ensembles d'entretoises est au moins partiellement recouvert par une membrane.

5. Dispositif médical de fermeture selon la revendication 1, **caractérisé en ce qu'**un bras d'au moins un ensemble d'entretoises comprend une section étendue de manière longitudinale (27b, 28b) provenant essentiellement de ladite section d'articulation, et ladite section étendue étant essentiellement parallèle aux bras dans la configuration d'introduction initiale, et
ladite section étendue se plie radialement vers l'extérieur lorsque le dispositif est comprimé.

6. Dispositif médical de fermeture selon la revendication 1, **caractérisé en ce que** le dispositif de fermeture comprend en outre un élément de blocage (30), qui comprend :
une première extrémité (32) avec au moins une dimension transversale externe supérieure à au moins une dimension transversale interne de la première partie d'extrémité (24) et une seconde extrémité (34) avec au moins une dimension transversale externe supérieure à au moins une dimension transversale interne de la seconde partie d'extrémité (26), et
une distance entre les première et seconde extrémités qui est inférieure à la longueur de l'élément tubulaire, de sorte que lorsque l'élément de blocage est positionné dans l'élément tubulaire, de sorte que la première extrémité de l'élément de blocage vient en butée contre la première partie d'extrémité, la seconde partie d'extrémité peut être forcée sur la seconde extrémité de sorte que le dispositif de fermeture est maintenu dans une configuration expansée dans laquelle les premier et le second ensembles d'entretoises se sont éloignés radialement dudit axe central longitudinal.

7. Dispositif médical de fermeture selon la revendication 1, **caractérisé en ce que** le dispositif de fermeture est au moins partiellement réalisé à partir d'un polymère synthétique résorbable.

8. Dispositif médical de fermeture selon la revendication 6, **caractérisé en ce que** ledit élément de blocage est réalisé à partir d'un premier matériau et ledit élément tubulaire est réalisé à partir d'un second matériau.

9. Dispositif médical de fermeture selon la revendication 1, **caractérisé en ce que** le dispositif de fermeture comprend un agent radio-opaque.

10. Dispositif médical de fermeture selon la revendication 1, **caractérisé en ce que** le dispositif de fermeture est adapté pour boucher un défaut au niveau du septum dans un coeur.

11. Dispositif médical de fermeture selon la revendication 1, **caractérisé en ce que** le dispositif de fermeture est adapté pour fermer une perforation dans une paroi de vaisseau.
